# EUROPEAN PATENT APPLICATION

(11) **EP 1 731 604 A1**
(43) Date of publication of application: **13.12.2006**
(21) Application number: 05721457.9
(22) Date of filing: 25.03.2005
(51) Int. Cl.: C12N 15/09, C12N 1/15, C12N 1/21, C12N 5/10, C12P 7/18, C12P 7/44

(54) **PROCESS FOR PRODUCING 1,3-PROPANEDIOL AND/OR 3-HYDROXYPROPIONIC ACID**

(30) Priority: 26.03.2004 JP 2004093417; 20.04.2004 JP 2004124524
(71) Applicant: NIPPON SHOKUBAI KAGAKU KOGYO CO. LTD., Osaka-shi, Osaka 541 (JP)
(72) Inventor: YASUDA, Shinzo, Ushiku-shi, Ibaraki 3001221 (JP); MUKOYAMA, Masaharu, Tsukubamirai-shi, Ibaraki 30022301 (JP); HORIKAWA, Hiroshi, Tsukuba-shi, Ibaraki 3050074 (JP); TORAYA, Tetsuo, Okayama-shi, Okayama 700002 (JP); MORITA, Hidetoshi, Tama-shi, Tokyo 2060011 (JP)
(74) Representative: Maschio, Antonio
(86) International application number: PCT/JP2005/005480
(87) International publication number: WO 2005/093060

(57) **Abstract**

This invention is intended to improve the efficiency of producing 1,3-propanediol from glycerol and to provide an industrially effective process for producing the same. Such process involves the use of a transformant comprising the gene encoding the large subunit of glycerol dehydratase and/or diol dehydratase, the gene encoding the medium subunit thereof, and the gene encoding the small subunit thereof, the gene encoding the large subunit of the reactivation factor for glycerol dehydratase and/or the reactivation factor for diol dehydratase and the gene encoding the small subunit thereof, the gene encoding aldehyde dehydrogenase, and the gene encoding 1,3-propanediol oxidoreductase and/or the gene encoding propanol dehydrogenase.

## Description

### Technical Field

The present invention relates to a transformant comprising the gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding aldehyde dehydrogenase; and the gene encoding 1,3-propanediol oxidoreductase and/or the gene encoding propanol dehydrogenase. The present invention also relates to knockout bacteria, from which the gene encoding glycerol dehydrogenase is knocked out. Further, the present invention relates to a method for producing 1,3-propanediol and/or 3-hydroxypropionic acid using such transformants or knockout bacteria.

### Background Art

1,3-Propanediol is a monomer that is used in the production of polyester fibers and of polyurethane and cyclic compounds. A variety of pathways for 1,3-propanediol synthesis are known. Examples of such methods include: a method wherein 1,3-propanediol is produced via catalytic conversion of ethylene oxide in the presence of phosphine, water, carbon monoxide, hydrogen, and acid; a method wherein 1,3-propanediol is produced via catalytic liquid-phase hydration of acrolein, followed by reduction; and a method wherein 1,3-propanediol is produced by allowing hydrocarbon (e.g., glycerol) to react in the presence of carbon monoxide, hydrogen, and a catalyst having a group VIII atom of the periodic table. However, conventional chemical synthesis has been disadvantageous in terms of cost and generation of wastes containing environmental pollutants.

In order to overcome such drawbacks, biological methods for producing 1,3-propanediol, i.e., methods that involve the use of microorganisms having enzymes for catalyzing the fermentation of glycerol to 1,3-propanediol, have been reported (see Patent Documents 1 to 6). Bacterial strains that can produce 1,3-propanediol from glycerol have been discovered in bacteria of genera such as *Citrobacter, Clostridium, Enterobacter, Salmonella, Klebsiella, Lactobacillus, Caloramator,* and *Listeria.*

In a biological system, glycerol is converted into 1,3-propanediol through a 2-stage enzyme catalytic reaction. At the first stage, glycerol dehydratase converts glycerol into 3-hydroxypropionaldehyde (3-HPA) and water (glycerol → 3-HPA+H₂O). At the second stage, 3-HPA is reduced to 1,3-propanediol by NAD⁺-dependent oxidoreductase (3-HPA+NADH+H⁺ → 1,3-propanediol +NAD⁺). 1,3-Propanediol is not further metabolized and it is consequently accumulated in a medium.

Production of 1,3-propanediol from glycerol in a biological system is generally carried out under anaerobic conditions using glycerol as a single carbon source in the absence of other exogenous reducing-equivalent receptors. Accordingly, a glycerol-related parallel path is activated, wherein glycerol is first oxidized to dihydroxyacetone (DHA) by NAD⁺- (or NADP⁺-)-bound glycerol dehydrogenase (i.e., glycerol + NAD⁺ → DHA + NADH + H). DHA is phosphorylated into dihydroxyacetone phosphate by DHA kinase and the product is utilized for biosynthesis and ATP generation.

According to conventional techniques for producing 1,3-propanediol involving the use of microorganisms, therefore, a half of the starting amount of glycerol is consumed in the parallel path, and the yield of the product is low in relation to the starting amount of glycerol. Thus, such techniques have been problematic in terms of efficiency and cost.

3-Hydroxypropionic acid and esters thereof are compounds that are useful as starting materials of aliphatic polyesters. Polyesters synthesized therefrom have drawn attention as biodegradable environmentally-friendly polyesters.

In general, 3-hydroxypropionic acid is produced by the addition of water to acrylic acid or by a reaction between ethylene chlorohydrin and sodium cyanate. Since the reaction whereby acrylic acid is hydrated is an equilibrium reaction, the response rate is disadvantageously limited. In the case of the reaction of ethylene chlorohydrin, use of virulent substances is required, and a step of hydrolysis is further required. In such a case, large quantities of sodium chloride and ammonium salts are disadvantageously generated.
Patent Document 1: WO 98/21339
Patent Document 2: WO 98/21341
Patent Document 3: US Patent No. 5,821,092
Patent Document 4: US Patent No. 5,254,467
Patent Document 5: US Patent No. 5,633,362
Patent Document 6: US Patent No. 5,686,276

### Disclosure of the Invention

The present invention is intended to improve the efficiently of the production of 1,3-propanediol from glycerol and to provide an industrially effective process for the production of the same.

The present inventors have conducted concentrated studies in order to attain the above objects. As a result, the present inventors discovered that two types of useful compounds could be effectively produced by bringing a transformant comprising the genes each encoding a subunit of glycerol dehydratase and/or diol dehydratase, the genes each encoding a subunit of the reactivation factor for glycerol dehydratase, the gene encoding aldehyde dehydrogenase, and the gene encoding 1,3-propanediol oxidoreductase and/or the gene encoding propanol dehydrogenase into contact with glycerol.

The present inventors also discovered that two types of useful compounds could be effectively produced by knocking out the gene encoding glycerol dehydrogenase of bacteria of the genera *Lactobacillus, Salmonella, Klebsiella, Listeria, Clostridium, Escherichia, Enterobacter, Caloramator, Acetobacterium, Brucella, Flavobacterium, Fusobacterium, Citrobacter,* and *Propionibacterium,* and bringing such bacteria into contact with glycerol.

Specifically, the present invention includes the following inventions.
(1) A transformant comprising the gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding aldehyde dehydrogenase; and the gene encoding 1,3-propanediol oxidoreductase and/or the gene encoding propanol dehydrogenase (propanediol oxidoreductase).
(2) The transformant according to (1), wherein the genes each encoding a subunit of glycerol dehydratase and/or diol dehydratase are derived from *Lactobacillus reuteri.*
(3) The transformant according to (2), wherein the gene encoding the large subunit of glycerol dehydratase encodes the following protein (a) or (b):
   (a) a protein comprising the amino acid sequence as shown in SEQ ID NO: 1 or 3; or
   (b) a protein comprising an amino acid sequence derived from the amino acid sequence as shown in SEQ ID NO: 1 or 3 by deletion, substitution, or addition of one or several amino acid residues and having glycerol dehydratase activity when expressed with the medium and small subunits of glycerol dehydratase;
      the gene encoding the medium subunit of glycerol dehydratase encodes the following protein (c) or (d):
   (c) a protein comprising the amino acid sequence as shown in SEQ ID NO: 5 or 7; or
   (d) a protein comprising an amino acid sequence derived from the amino acid sequence as shown in SEQ ID NO: 5 or 7 by deletion, substitution, or addition of one or several amino acid residues and having glycerol dehydratase activity when expressed with the large and small subunits of glycerol dehydratase; and
      the gene encoding the small subunit of glycerol dehydratase encodes the following protein (e) or (f):
   (e) a protein comprising the amino acid sequence as shown in SEQ ID NO: 9 or 11; or
   (f) a protein comprising an amino acid sequence derived from the amino acid sequence as shown in SEQ ID NO: 9 or 11 by deletion, substitution, or addition of one or several amino acid residues and having glycerol dehydratase activity when expressed with the large and medium subunits of glycerol dehydratase.
(4) The transformant according to (2), wherein the gene encoding the large subunit of glycerol dehydratase comprises the following DNA (a) or (b):
   (a) DNA comprising the nucleotide sequence as shown in SEQ ID NO: 2 or 4; or
   (b) DNA hybridizing under stringent conditions with DNA comprising a nucleotide sequence complementary to DNA comprising part of or the entire nucleotide sequence as shown in SEQ ID NO: 2 or 4 and encoding a protein having glycerol dehydratase activity when expressed with the medium and small subunits of glycerol dehydratase;
      the gene encoding the medium subunit of glycerol dehydratase comprising the following DNA (c) or (d):
   (c) DNA comprising the nucleotide sequence as shown in SEQ ID NO: 6 or 8; or
   (d) DNA hybridizing under stringent conditions with DNA comprising a nucleotide sequence complementary to DNA comprising part of or the entire nucleotide sequence as shown in SEQ ID NO: 6 or 8 and encoding a protein having glycerol dehydratase activity when expressed with the large and small subunits of glycerol dehydratase; and
      the gene encoding the small subunit of glycerol dehydratase comprises the following DNA (e) or (f):
   (e) DNA comprising the nucleotide sequence as shown in SEQ ID NO: 10 or 12; or
   (f) DNA hybridizing under stringent conditions with DNA comprising a nucleotide sequence complementary to DNA comprising part of or the entire nucleotide sequence as shown in SEQ ID NO: 10 or 12 and encoding a protein having glycerol dehydratase activity when expressed with the large and medium subunits of glycerol dehydratase.
(5) The transformant according to any of (1) to (4), which comprises the gene encoding propanol dehydrogenase (propanediol oxidoreductase), such gene being derived from *Lactobacillus reuteri.*
(6) The transformant according to (5), wherein the gene encoding propanol dehydrogenase (propanediol oxidoreductase) encodes the following protein (a) or (b):
   (a) a protein comprising the amino acid sequence as shown in SEQ ID NO: 13 or 15; or
   (b) a protein comprising an amino acid sequence derived from the amino acid sequence as shown in SEQ ID NO: 13 or 15 by deletion, substitution, or addition of one or several amino acid residues and having propanol dehydrogenase (propanediol oxidoreductase) activity.
(7) The transformant according to (5), wherein the gene encoding propanol dehydrogenase (propanediol oxidoreductase) comprises the following DNA (a) or (b):
   (a) DNA comprising the nucleotide sequence as shown in SEQ ID NO: 14 or 16; or
   (b) DNA hybridizing under stringent conditions with DNA comprising a nucleotide sequence complementary to DNA comprising part of or the entire nucleotide sequence as shown in SEQ ID NO: 14 or 16 and encoding a protein having propanol dehydrogenase (propanediol oxidoreductase) activity.
(8) The transformant according to any of (1) to (7), which comprises the gene encoding 1,3-propanediol oxidoreductase, such gene being derived from *Lactobacillus reuteri.*
(9) The transformant according to (8), wherein the gene encoding 1,3-propanediol oxidoreductase encodes the following protein (a) or (b):
   (a) a protein comprising the amino acid sequence as shown in SEQ ID NO: 17; or
   (b) a protein comprising an amino acid sequence derived from the amino acid sequence as shown in SEQ ID NO: 17 by deletion, substitution, or addition of one or several amino acid residues and having 1,3-propanediol oxidoreductase activity.
(10) The transformant according to (8), wherein the gene encoding 1,3-propanediol oxidoreductase comprises the following DNA (a) or (b):
   (a) DNA comprising the nucleotide sequence as shown in SEQ ID NO: 18; or
   (b) DNA hybridizing under stringent conditions with DNA comprising a nucleotide sequence complementary to DNA comprising part of or the entire nucleotide sequence as shown in SEQ ID NO: 18 and encoding a protein having 1,3-propanediol oxidoreductase activity.
(11) The transformant according to any of (1) to (10), wherein the genes each encoding a subunit of the reactivation factor for glycerol dehydratase and/or the reactivation factor for diol dehydratase are derived from *Lactobacillus reuteri.*
(12) The transformant according to (11), wherein the gene encoding the large subunit of the reactivation factor for glycerol dehydratase encodes the following protein (a) or (b):
   (a) a protein comprising the amino acid sequence as shown in SEQ ID NO: 19 or 21; or
   (b) a protein comprising an amino acid sequence derived from the amino acid sequence as shown in SEQ ID NO: 19 or 21 by deletion, substitution, or addition of one or several amino acid residues and having the activity of the reactivation factor for glycerol dehydratase when expressed with the small subunit of the reactivation factor for glycerol dehydratase, and
      the gene encoding the small subunit of the reactivation factor for glycerol dehydratase encodes the following protein (c) or (d):
   (c) a protein comprising the amino acid sequence as shown in SEQ ID NO: 23 or 25; or
   (d) a protein comprising an amino acid sequence derived from the amino acid sequence as shown in SEQ ID NO: 23 or 25 by deletion, substitution, or addition of one or several amino acid residues and having the activity of the reactivation factor for glycerol dehydratase when expressed with the large subunit of the reactivation factor for glycerol dehydratase.
(13) The transformant according to (11), wherein the gene encoding the large subunit of the reactivation factor for glycerol dehydratase comprises the following DNA (a) or (b):
   (a) DNA comprising the nucleotide sequence as shown in SEQ ID NO: 20 or 22; or
   (b) DNA hybridizing under stringent conditions with DNA comprising a nucleotide sequence complementary to DNA comprising part of or the entire nucleotide sequence as shown in SEQ ID NO: SEQ ID NO: 20 or 22 and encoding a protein having the activity of the reactivation factor for glycerol dehydratase when expressed with the small subunit of the reactivation factor for glycerol dehydratase, and
      the gene encoding the small subunit of the reactivation factor for glycerol dehydratase comprises the following DNA (c) or (d):
   (c) DNA comprising the nucleotide sequence as shown in SEQ ID NO: 24 or 26; or
   (d) DNA hybridizing under stringent conditions with DNA comprising a nucleotide sequence complementary to DNA comprising part of or the entire nucleotide sequence as shown in SEQ ID NO: SEQ ID NO: 24 or 26 and encoding a protein having the reactivation factor for glycerol dehydratase when expressed with the large subunit of the reactivation factor for glycerol dehydratase.
(14) A method for producing 1,3-propanediol and 3-hydroxypropionic acid comprising culturing the transformant according to any of (1) to (13) in the presence of glycerol.
(15) Knockout bacteria, which are obtained by knocking out the gene encoding glycerol dehydrogenase from bacteria of the genera *Lactobacillus, Salmonella, Klebsiella, Listeria, Clostridium, Escherichia, Enterobacter, Caloramator, Acetobacterium, Brucella, Flavobacterium, Fusobacterium, Citrobacter,* or *Propionibacterium.*
(16) Knockout bacteria, which are obtained by knocking out the gene encoding glycerol dehydrogenase from bacteria having the pdu operon and the gene encoding phosphotransacylase.
(17) A method for producing 1,3-propanediol and/or 3-hydroxypropionic acid by bringing the bacteria of (15) or (16) into contact with glycerol.
(18) A transformant comprising the gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding propionaldehyde dehydrogenase; the gene encoding phosphotransacylase; the gene encoding propionate kinase; and the gene encoding 1,3-propanediol oxidoreductase and/or the gene encoding propanol dehydrogenase (propanediol oxidoreductase) and not comprising any gene encoding glycerol dehydrogenase.
(19) The transformant according to (18) having the pdu operon and not comprising any gene encoding glycerol dehydrogenase.
(20) The transformant according to (18) or (19), wherein the gene encoding propionaldehyde dehydrogenase encodes the following protein (a) or (b):
   (a) a protein comprising the amino acid sequence as shown in SEQ ID NO: 41; or
   (b) a protein comprising an amino acid sequence derived from the amino acid sequence as shown in SEQ ID NO: 41 by deletion, substitution, or addition of one or several amino acid residues and having propionaldehyde dehydrogenase activity.
(21) The transformant according to (18) or (19), wherein the gene encoding propionaldehyde dehydrogenase comprises the following DNA (a) or (b):
   (a) DNA comprising the nucleotide sequence as shown in SEQ ID NO: 42; or
   (b) DNA hybridizing under stringent conditions with DNA comprising a nucleotide sequence complementary to DNA comprising part of or the entire nucleotide sequence as shown in SEQ ID NO: 42 and encoding a protein having the propionaldehyde dehydrogenase activity.
(22) The transformant according to (18) or (19), wherein the gene encoding propionate kinase encodes the following protein (a) or (b):
   (a) a protein comprising the amino acid sequence as shown in SEQ ID NO: 43; or
   (b) a protein comprising an amino acid sequence derived from the amino acid sequence as shown in SEQ ID NO: 43 by deletion, substitution, or addition of one or several amino acid residues and having propionate kinase activity.
(23) The transformant according to (18) or (19), wherein the gene encoding propionate kinase comprises the following DNA (a) or (b):
   (a) DNA comprising the nucleotide sequence as shown in SEQ ID NO: 44; or
   (b) DNA hybridizing under stringent conditions with DNA comprising a nucleotide sequence complementary to DNA comprising part of or the entire nucleotide sequence as shown in SEQ ID NO: 44 and encoding a protein having the propionate kinase activity.
(24) A method for producing 1,3-propanediol and/or 3-hydroxypropionic acid by bringing the transformant according to any of (18) to (23) into contact with glycerol.

According to the present invention, loss of starting glycerol used when producing 1,3-propanediol from glycerol can be reduced and 3-hydroxypropionic acid can also be produced in addition to 1,3-propanediol. Since bacteria used for such production can be effectively cultured, 1,3-propanediol and 3-hydroxypropionic acid can be produced with higher efficiency.

### Brief Description of the Drawings

Fig. 1 shows the structure of the pdu operon.
Fig. 2 shows an embodiment of a mechanism for producing 1,3-propanediol and 3-hydroxypropionic acid from glycerol.

### Preferred Embodiments of the Invention

Hereafter, the present invention is described in detail.

The first aspect of the present invention relates to a transformant comprising the gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding aldehyde dehydrogenase; and the gene encoding 1,3-propanediol oxidoreductase and/or the gene encoding propanol dehydrogenase.

The transformant according to the first aspect comprises the gene that encodes a protein having enzyme activity of catalyzing the reaction whereby glycerol is dehydrated and converted into 3-hydroxypropionaldehyde and water. Examples of such proteins include glycerol dehydratase and diol dehydratase. Glycerol dehydratase and diol dehydratase are each composed of 3 types of subunits, i.e., the large, medium, and small subunits. Examples of the transformant according to the present invention include those comprising the genes each encoding such 3 types of subunits of glycerol dehydratase, those comprising the genes each encoding such 3 types of subunits of diol dehydratase, and those comprising both the genes each encoding such 3 types of subunits of glycerol dehydratase and the genes each encoding such 3 types of subunits of diol dehydratase.

Known genes each encoding a subunit of glycerol dehydratase or diol dehydratase can be employed. For example, genes derived from bacteria of the genera *Lactobacillus, Citrobacter, Clostridium, Klebsiella, Enterobacter, Caloramator, Salmonella,* and *Listeria* genera can be employed. In the present invention, the genes each encoding a subunit of glycerol dehydratase and/or diol dehydratase derived from the bacteria of the genus *Lactobacillus* are preferable. The genes each encoding a subunit of glycerol dehydratase and/or diol dehydratase derived from *Lactobacillus reuteri* are more preferable, and the genes each encoding a subunit of glycerol dehydratase and/or diol dehydratase derived from the *Lactobacillus reuteri* JCM1112 strain and the *Lactobacillus reuteri* ATCC 53608 strain are further preferable.

SEQ ID NOs: 1 and 3 show the amino acid sequences of the large subunit of glycerol dehydratase derived from *Lactobacillus reuteri.* SEQ ID NOs: 5 and 7 show the amino acid sequences of the medium subunit thereof. SEQ ID NOs: 9 and 11 show the amino acid sequences of the small subunit thereof. SEQ ID NOs: 2 and 4 show the nucleotide sequences of the gene encoding the large subunit of glycerol dehydratase derived from *Lactobacillus reuteri.* SEQ ID NOs: 6 and 8 show the nucleotide sequences of the gene encoding the medium subunit thereof. SEQ ID NOs: 10 and 12 show the nucleotide sequences of the gene encoding the small subunit thereof.

Amino acid sequences of proteins may include mutations such as deletion, substitution, or addition of one or several amino acid residues as long as the proteins have glycerol dehydratase activity when expressed with the two other types of subunits.

The present invention also includes the use of a gene that hybridizes under stringent conditions with a sequence complementary to DNA comprising part of or the entire nucleotide sequence as shown in each SEQ ID NO and that encodes a protein having glycerol dehydratase activity when expressed with the other two types of subunits.

The genes each encoding a subunit may be introduced into the same or different vectors to carry out transformation, as long as such genes are expressed in the same host. It is preferable that three types of subunits be derived from the same species or strain.

The transformant according to the first aspect comprises the gene encoding propanol dehydrogenase, the gene encoding 1,3-propanediol oxidoreductase, or both such genes.

In the present invention, the term "propanol dehydrogenase" (which also may be referred to as "propanediol oxidoreductase") is used in the general sense with which it is used in the art. That is, it refers to a protein having enzyme activity that can catalyze a reaction whereby 3-hydroxypropionaldehyde is reduced and converted into propanediol.

Known genes encoding propanol dehydrogenase can be employed. For example, genes derived from bacteria of the genera *Lactobacillus, Citrobacter, Clostridium, Klebsiella, Enterobacter, Caloramator, Salmonella,* and *Listeria* can be employed. In the present invention, propanol dehydrogenase genes derived from the bacteria of the genus *Lactobacillus* are preferable, propanol dehydrogenase genes derived from *Lactobacillus reuteri* are more preferable, and propanol dehydrogenase genes derived from the *Lactobacillus reuteri* JCM1112 strain and the *Lactobacillus reuteri* ATCC 53608 strain are further preferable.

SEQ ID NOs: 13 and 15 show the amino acid sequences of propanol dehydrogenase derived from *Lactobacillus reuteri.* SEQ ID NOs: 14 and 16 show the nucleotide sequences of propanol dehydrogenase genes derived from *Lactobacillus reuteri.* As long as the proteins comprising such amino acid sequences have propanol dehydrogenase activity, the amino acid sequence as shown in SEQ ID NO: 13 or 15 may include mutations such as deletion, substitution, or addition of one or several amino acid residues.

The present invention also includes the use of a gene that hybridizes under stringent conditions with a sequence complementary to DNA comprising part of or the entire nucleotide sequence of DNA comprising a sequence as shown in SEQ ID NO: 14 or 16 and that encodes a protein having propanol dehydrogenase activity.

In the present invention, the term "1,3-propanediol oxidoreductase" is used in the general sense with which it is used in the art. That is, it refers to a protein having enzyme activity that can catalyze a reaction whereby 3-hydroxypropionaldehyde is reduced and converted into 1,3-propanediol.

Known genes encoding 1,3-propanediol oxidoreductase can be employed. For example, genes derived from bacteria of the genera *Lactobacillus, Citrobacter, Clostridium, Klebsiella, Enterobacter, Caloramator, Salmonella,* and *Listeria* can be employed. In the present invention, the 1,3-propanediol oxidoreductase genes derived from bacteria of the genus *Lactobacillus* are preferable, the 1,3-propanediol oxidoreductase genes derived from *Lactobacillus reuteri* are more preferable, and the 1,3-propanediol oxidoreductase genes derived from the *Lactobacillus reuteri* JCM1112 strain and the *Lactobacillus reuteri* ATCC 53608 strains are further preferable.

SEQ ID NO: 17 shows the amino acid sequence of the 1,3-propanediol oxidoreductase gene derived from *Lactobacillus reuteri* and SEQ ID NO: 18 shows the nucleotide sequence of the 1,3-propanediol oxidoreductase gene derived from *Lactobacillus reuteri.* As long as proteins comprising such amino acid sequences have 1,3-propanediol oxidoreductase activity, the amino acid sequence as shown in SEQ ID NO: 17 may include mutations such as deletion, substitution, or addition of one or several amino acid residues.

The present invention also includes the use of a gene that hybridizes under stringent conditions with a sequence complementary to DNA comprising part of or the entire nucleotide sequence of DNA comprising the nucleotide sequence as shown in SEQ ID NO: 18 and that encodes a protein having 1,3-propanediol oxidoreductase activity.

The transformant according to the first aspect of the present invention comprises the gene encoding a protein that replaces coenzyme B12 located at the reaction center of glycerol dehydratase or diol dehydratase, which had been deactivated via catalysis of conversion of glycerol into 3-hydroxypropionaldehyde and water, and regains its activity. Examples of such proteins include the reactivation factor for glycerol dehydratase and the reactivation factor for diol dehydratase. The reactivation factor for glycerol dehydratase and the reactivation factor for diol dehydratase are each composed of two types of subunits, i.e., a large subunit and a small subunit. Examples of the transformant of the present invention include those comprising the genes each encoding two types of subunits of the reactivation factor for glycerol dehydrogenase, those comprising the genes each encoding two types of subunits of the reactivation factor for diol dehydratase, and those comprising the genes each encoding two types of subunits of the reactivation factor for glycerol dehydratase and the genes each encoding two types of subunits of the reactivation factor for diol dehydratase.

Any gene encoding the reactivation factor can be employed without particular limitation, as long as such genes have equivalent functions. Examples of the reactivation factor for glycerol dehydratase include those disclosed in WO 98/21341; Daniel et al., J. Bacteriol., 177, 2151, 1995; Toraya and Mori, J. Biol. Chem., 274, 3372(1999); and Tobimatsu et al., J. Bacteriol. 181, 4110, 1999.

Genes each encoding a subunit of the reactivation factor for glycerol dehydratase or the reactivation factor for diol dehydratase include those existing in a group of genes referred to as gdh regulons and pdu operons possessed by a group of bacteria that can assimilate glycerol under anaerobic conditions, in general. Examples thereof include gdrA, gdrB, pduG, pduH, ddrA, ddrB, dhaF, dhaG, orfZ, and orfY.

Known genes each encoding a subunit of the reactivation factor for glycerol dehydratase or the reactivation factor for diol dehydratase can be employed. Examples thereof include genes derived from bacteria of the genera *Lactobacillus, Citrobacter, Clostridium, Klebsiella, Enterobacter, Caloramator, Salmonella,* and *Listeria.* In the present invention, the genes each encoding a subunit of the reactivation factor for glycerol dehydratase and/or the reactivation factor for diol dehydratase derived from bacteria of the genus *Lactobacillus* are preferable, the genes each encoding a subunit of the reactivation factor for glycerol dehydratase and/or the reactivation factor for diol dehydratase derived from *Lactobacillus reuteri* are more preferable, and the genes each encoding a subunit of the reactivation factor for glycerol dehydratase and/or the reactivation factor for diol dehydratase derived from the *Lactobacillus reuteri* JCM1112 strain and the *Lactobacillus reuteri* ATCC 53608 strain are further preferable.

Preferably, a transformant comprising genes each encoding 3 types of subunits of glycerol dehydratase comprises at least genes each encoding 2 types of subunits of the reactivation factor for glycerol dehydratase and a transformant comprising genes each encoding 3 types of subunits of diol dehydratase comprises at least genes each encoding 2 types of subunits of the reactivation factor for diol dehydratase.

SEQ ID NOs: 19 and 21 show the amino acid sequences of the large subunit of the reactivation factor for glycerol dehydratase derived from *Lactobacillus reuteri.* SEQ ID NOs: 23 and 25 show the amino acid sequences of the small subunit thereof. SEQ ID NOs: 20 and 22 show the nucleotide sequences of the genes encoding the large subunit of glycerol dehydratase derived from *Lactobacillus reuteri.* SEQ ID NOs: 24 and 26 show the nucleotide sequences of the gene encoding the small subunit thereof.

As long as a protein comprising each such amino acid sequence has the activity of the reactivation factor for glycerol dehydratase when expressed with another subunit, an amino acid sequence may include mutations such as deletion, substitution, or addition of one or several amino acid residues.

The present invention also includes the use of a gene that hybridizes under stringent conditions with a sequence complementary to DNA comprising part of or the entire DNA comprising the nucleotide sequence as shown in each SEQ ID NO and that encodes a protein having the activity of the reactivation factor for glycerol dehydratase when expressed with another subunit.

The genes each encoding a subunit may be introduced into the same or different vectors to carry out transformation, as long as such genes are expressed in the same host. It is preferable that three types of subunits be derived from the same species or strain.

An amino acid sequence derived from the amino acid sequence as shown in a given SEQ ID NO by deletion, substitution, addition of one or several amino acid residues may involve deletion, addition, or substitution of 1, preferably 10 to 20, more preferably 5 to 10, and further preferably 2 or 3 amino acid residues in the amino acid sequence as shown in a given SEQ ID NO.

Under stringent conditions, a specific hybrid is formed and a nonspecific hybrid is not formed. That is, DNA having high homology (homology of 90% or higher, and preferably 95% or higher) to a given gene hybridizes under such conditions. More specifically, such conditions can be realized by conducting hybridization in the presence of 0.5 to 1 M NaCl at 42°C to 68°C, in the presence of 50% formamide at 42°C, or in an aqueous solution at 65°C to 68°C, and then washing the filter using a 0.1- to 2-fold saline sodium citrate (SSC) solution at a temperature between room temperature and 68°C.

The term "part of the sequence" refers to a nucleotide sequence of DNA comprising part of a nucleotide sequence of a given gene, which has a sufficient nucleotide sequence length to conduct hybridization under stringent conditions. For example, such sequence comprises at least 50, preferably at least 100, and more preferably at least 200 nucleotides.

Mutation can be introduced into a gene in accordance with conventional techniques such as the Kunkel method or the Gapped duplex method, or via techniques in accordance therewith, with the use of a mutagenesis kit utilizing site-specific mutagenesis (e.g., Mutan-K (TAKARA) or Mutan-G (TAKARA)) or the LA PCR in vitro Mutagenesis Series Kit (TAKARA). After the nucleotide sequence has been determined by the above technique, the gene of the present invention can be obtained via chemical synthesis, PCR using chromosome DNA as a template, or hybridization involving the use of a DNA fragment containing such nucleotide sequence as a probe.

In the present invention, the term "aldehyde dehydrogenase" is used in the general sense with which it is used in the art. Specifically, it refers to a protein having enzyme activity of oxidizing aldehyde and generating carboxylic acid or acyl group.

Known genes encoding aldehyde dehydrogenase can be employed. For example, genes derived from bacteria of the genera *Alcaligenes, Aspergillus, Bacillus, Candida, Chromobacterium, Clostridium, Corynebacterium, Escherichia, Lactobacillus, Lactococcus, OceanoBacillus, Pichia, Pseudomonas, Rhizobium, Rhodobacter, Rhodococcus, Saccharomyces, Salmonella, Sulfolobus,* and *Thermotoga* can be used.

Transformants can be obtained by ligating the 4 aforementioned types of genes or parts thereof to an adequate vector and introducing the resulting recombinant vector into a host so as to allow the gene of the present invention to express therein. The term "part" refers to a portion of a given gene that can express a protein encoded by such gene when introduced into a host.

A technique of obtaining a gene of interest from a bacteria genome is known in the field of molecular biology. When the gene sequence is known, for example, an adequate genome library is prepared by restriction endonuclease digestion, and the gene of interest can be screened for with the use of a probe complementary to the gene sequence of interest. Upon isolation of the sequence, DNA thereof may be amplified via a standard amplification technique such as polymerase chain reaction (PCR, US Patent No. 4,683,202) to obtain DNA in an adequate amount for transformation.

The genes each encoding a subunit of glycerol dehydratase and/or diol dehydratase, the 1,3-propanediol oxidoreductase gene, the propanol dehydrogenase gene, the genes each encoding a subunit of the reactivation factor for glycerol dehydratase and/or the reactivation factor for diol dehydratase, and the aldehyde dehydrogenase genes may be separately introduced into a plurality of vectors to carry out transformation. Alternatively, several types of genes may be introduced into a single vector to carry out transformation.

Vectors for introducing genes are not particularly limited, as long as such vectors can replicate the genes of interest in host cells. Examples thereof include plasmid DNA, phage DNA, and cosmid DNA. Examples of plasmid DNA include pBR322, pSC101, pUC18, pUC19, pUC118, pUC119, pACYC117, pBluescript II SK(+), pETDuet-1, and pACYCDuet-1. Examples of phage DNA include λgt10, Charon 4A, EMBL-, M13mp18, and M13mp19.

Any hosts can be used without particular limitation, as long as the genes of interest can be expressed therein. Examples of such hosts include bacteria of the genus *Ralstonia* such as *Ralstonia eutropha,* bacteria of the genus *Pseudomonas* such as *Pseudomonas putida,* bacteria of the genus *Bacillus* such as *Bacillus subtilis,* bacteria of the genus *Escherichia* such as *E. coli,* yeast of the genus *Saccharomyces* such as *Saccharomyces cerevisiae,* yeast of the genus *Candida* such as *Candida maltosa,* animal cells such as COS cells, CHO cells, mouse L cells, rat GH3 cells, and human FL cells, and insect cells such as SF9 cells.

It is preferable to use a host cell, wherein glycerol dehydrogenase is not expressed; i.e., a cell that does not contain the glycerol dehydrogenase gene or a cell from which the glycerol dehydrogenase gene is knocked out. Use of such cell can block the pathway whereby glycerol is oxidized and converted into dihydroxyacetone. Thus, 1,3-propanediol and 3-hydroxypropionic acid can be produced with higher yield.

The term "cell from which the glycerol dehydrogenase gene is knocked out" refers to a cell wherein the glycerol dehydrogenase gene has been disrupted and thus cannot be expressed. Specifically, such cell is prepared by disrupting the glycerol dehydrogenase gene in the cell by a method wherein the glycerol dehydrogenase gene in the cell is designated as the target gene and a vector that induces homologous recombination (i.e., a targeting vector) at any position in such target gene is used to disrupt the target gene (i.e., the gene targeting method), a method wherein a trap vector (i.e., a reporter gene not comprising any promoter) is inserted into any position in the target gene to disrupt and deactivate the target gene (i.e., the gene trap method), or combinations of such common techniques known in the art, which are often employed when producing knockout cells or transgenic animals (including knockout animals). The positions at which homologous substitution is induced to take place or at which a trap vector is to be inserted are not particularly limited, as long as mutation at such position can result in elimination of the glycerol dehydrogenase gene expression. Preferably, a transcriptional control region, and more preferably the second exon, is substituted. Examples of other methods of knocking out the glycerol dehydrogenase gene include a method wherein a vector expressing antisense cDNA of the glycerol dehydrogenase gene is introduced into cells and a method wherein a vector expressing double-strand RNA of the glycerol dehydrogenase gene is introduced into cells. Examples of such vectors include virus and plasmid vectors. Such vectors can be prepared in accordance with conventional genetic engineering techniques, such as the method described in fundamental textbooks such as Molecular cloning 2nd Ed., Cold Spring Harbor Laboratory Press, 1989. A commercialized vector may be cleaved with any restriction enzyme, and a gene of interest or the like may be incorporated therein to prepare a semisynthetic vector.

Whether or not the glycerol dehydrogenase gene is knocked out can be determined in the following manner. That is, the cells into which the vector has been introduced are subjected to Southern blotting to confirm that homologous recombination has adequately occurred. Alternatively, a drug resistant gene that is not present in a host cell is introduced into a targeting vector and a drug resistant cell is selected. It can also be determined in the following manner: after the introduction of disruption, PCR is carried out using the genome of the selected cell, bacteria, bacterial culture solution, or the like as a template and using the forward and reverse primers of the glycerol dehydrogenase gene to be disrupted; and confirm amplification of the DNA fragment to be obtained by combination of the glycerol dehydrogenase and the disruption introducing site.. The resulting DNA fragment may be subjected to cloning for sequence analysis. Knock out can also be determined by confirming that dihydroxyacetone is not generated.

When bacterial host cells such as *E*. *coli* are used, it is preferable that a recombinant vector be capable of autonomous replication in such host cells and that a recombinant vector comprise a promoter, target DNA, and a terminator sequence. Expression vectors are replicated and maintained in a wide variety of host cells. Examples thereof include pLA2917 (ATCC 37355) originating from the RK2 replication origin and pJRD215 (ATCC 37533) originating from the RSF1010 replication origin.

Any promoter can be employed as long as it can express a gene of interest in a host cell. Examples thereof include E. *coli* or phage-derived promoters, such as trp promoter, lac promoter, PL promoter, PR promoter, and T7 promoter. A recombinant vector may be introduced into bacteria by any method without particular limitation. Examples of such methods include a method involving the use of calcium ions (Current Protocols in Molecular Biology, 1, 181, 1994) or electroporation.

When yeast host cells are used, YEp13 or YCp50 expression vectors can be used, for example. Examples of promoters include gal 1 promoter, gal 10 promoter, heat shock protein promoter, and GAP promoter. A recombinant vector may be introduced into a yeast cell by any method without particular limitation. Examples of such methods include electroporation, spheroplast, (Proc. Natl. Acad. Sci. USA, 84, 192, 9-1933, 1978), and the lithium acetate method (J. Bacteriol., 153, 163-168, 1983).

When animal host cells are used, pcDNAI or pcDNAI/Amp (Invitrogen) expression vectors may be used, for example. Examples of promoters include SRa promoter, SV40 promoter, and CMV promoter. A recombinant vector may be introduced into animal cells by any method without particular limitation. Examples of such methods include electroporation, the calcium phosphate method, and lipofection.

Techniques for isolating genes and preparing transformants are described in Sambrook, J. et al., Molecular Cloning: A Laboratory Manual, Second Edition, 1989, Cold Spring Harbor Laboratory Press.

The second aspect of the present invention relates to knockout bacteria, which is obtained by knocking out the gene encoding glycerol dehydrogenase from bacteria of the genera *Lactobacillus, Salmonella, Klebsiella, Listeria, Clostridium, Escherichia, Enterobacter, Caloramator, Acetobacterium, Brucella, Flavobacterium, Fusobacterium, Citrobacter,* or *Propionibacterium.* In the present invention, the bacteria of the genera *Lactobacillus, Salmonella, Klebsiella, Listeria, Clostridium, Escherichia, Enterobacter, Caloramator, Acetobacterium, Brucella, Flavobacterium, Fusobacterium, Citrobacter,* and *Propionibacterium* are not particularly limited, as long as they comprise the gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding propionaldehyde dehydrogenase; the gene encoding phosphotransacylase; the gene encoding propionate kinase; and the gene encoding 1,3-propanediol oxidoreductase and/or the gene encoding propanol dehydrogenase.

In the second aspect of the present invention, bacteria having a system of coenzyme B12 synthesis are preferable. Examples thereof include bacteria of the genera *Lactobacillus, Salmonella, Klebsiella, Brucella, Fusobacterium,* and *Propionibacterium.*

Examples of bacteria of the genus *Lactobacillus* include *Lactobacillus reuteri, Lactobacillus brevis, Lactobacillus collinoides, Lactobacillus hilgardii, Lactobacillus diolivorans, Lactobacillus buchneri, Lactobacillus fermentum, Lactobacillus gasseri, Lactobacillus helveticus, Lactobacillus plantarum, Lactobacillus johnsonii,* and *Lactobacillus yamanashiensis.*

Examples of bacteria of the genus *Salmonella* include *Salmonella enterica, Salmonella enteritidis, Salmonella typhi,* and *Salmonella typhimurium.* Examples of bacteria of the genus *Klebsiella* include *Klebsiella aerogenes, Klebsiella oxytoca, Klebsiella pneumoniae, Klebsiella atlantae, Klebsiella edwardsii, Klebsiella mobilis, Klebsiella ozaenae, Klebsiella planticola, Klebsiella rhinoscleromatis, Klebsiella rubiacearum,* and *Klebsiella terrigena.* Examples of bacteria of the genus *Listeria* include *Listeria denitrificans, Listeria grayi, Listeria innocua, Listeria ivanovii, Listeria monocytogenes, Listeria murrayi, Listeria seeligeri,* and *Listeria welshimeri.* Examples of bacteria of the genus *Clostridium* include *Clostridium acetobutylicum, Clostridium butyricum, Clostridium pasteurianum, Clostridium paraperfringens, Clostridium perfringens, Clostridium glycolicum,* and *Clostridium diffcile.* Examples of bacteria of the genus *Escherichia* include *Escherichia blattae, Escherichia hermannii, Escherichia vulneris,* and *Escherichia freundii.* Examples of bacteria of the genus *Enterobacter* include *Enterobacter aerogenes* and *Enterobacter agglomerans.* Examples of bacteria of the genus *Caloramator* include *Caloramator coolhaasii, Caloramator fervidus, Caloramator indicus, Caloramator proteoclasticus, Caloramator uzoniensis,* and *Caloramator viterbiensis.* An example of bacteria of the genus *Acetobacterium* is *Acetobacterium sp.,* an example of bacteria of the genus *Brucella* is *Brucella melitensis,* an example of bacteria of the genus *Flavobacterium* is *Flavobacterium sp.,* an example of bacteria of the genus *Fusobacterium* is *Fusobacterium nucleatum,* and an example of bacteria of the genus *Citrobacter* is *Citrobacter freundii.*

Examples of bacteria of the genus *Propionibacterium* include *Propionibacterium acidipropionici, Propionibacterium acnes, Propionibacterium australiense, Propionibacterium avidum, Propionibacterium cyclohexanicum, Propionibacterium granulosum, Propionibacterium jensenii, Propionibacterium microaephilum, Propionibacterium propionicum, Propionibacterium thoenii,* and *Propionibacterium freudenreichii.*

In the second aspect of the present invention, bacteria of the genus *Lactobacillus* are preferably used, *Lactobacillus reuteri* is more preferably used and the *Lactobacillus reuteri* JCM1112 strain is particularly preferably used. Also, bacteria of the genus *Klebsiella* are preferably used, *Klebsiella pneumoniae* and *Klebsiella oxytoca* are more preferably used and the *Klebsiella pneumoniae* ATCC 25955 strain and the *Klebsiella oxytoca* ATCC 8724 strain are particularly preferably used.

Bacteria that are used in the second aspect of the present invention are knockout bacteria of the aforementioned bacteria, from which the gene encoding glycerol dehydrogenase is knocked out. Knocking out of such gene can block the pathway whereby glycerol is oxidized and converted into dihydroxyacetone. Thus, 1,3-propanediol and/or 3-hydroxypropionic acid can be produced with higher yields.

The gene encoding glycerol dehydrogenase being knocked out means that the glycerol dehydrogenase gene is disrupted and thus cannot be expressed. The knockout bacteria according to this aspect are as described with respect to the first aspect of the present invention.

In the second aspect of the present invention, bacteria of the genera *Lactobacillus, Salmonella, Klebsiella, Listeria, Clostridium, Escherichia, Enterobacter, Caloramator, Acetobacterium, Brucella, Flavobacterium, Fusobacterium, Citrobacter,* and *Propionibacterium* each preferably comprise the pdu operon and the gene encoding phosphotransacylase, and the gene encoding glycerol dehydrogenase are preferably knocked out.

In addition to bacteria of the genera *Lactobacillus, Salmonella, Klebsiella, Listeria, Clostridium, Escherichia, Enterobacter, Caloramator, Acetobacterium, Brucella, Flavobacterium, Fusobacterium, Citrobacter,* and *Propionibacterium,* bacteria comprising the pdu operon and the gene encoding phosphotransacylase, from which the gene encoding glycerol dehydrogenase is knocked out, are also within the scope of this aspect.

The pdu operon comprises a gene encoding a protein of the polyhedral body and has function of retaining aldehyde derived from 1,2-diols such as glycerin for a given period of time in such polyhedral body, catalyzing reactions to convert aldehyde into acid or alcohol in the polyhedral body, on the membrane of polyhedral body, or in the vicinity thereof, and reducing direct adverse effects of aldehyde on cells.

Accordingly, microorganisms comprising pdu operons form polyhedral bodies in the cells, incorporate diols in such polyhedral bodies, and retain a given amount of aldehyde resulting from dehydration to prevent adverse effects on cell growth. Thus, aldehyde oxidation or reduction is considered to take place in the polyhedral bodies, on the membranes of polyhedral bodies, or in the vicinity thereof. The bacteria of the present invention preferably comprise the open reading frame (ORF) encoding the protein for the formation of polyhedral body and other ORFs contained in the pdu operon as well as in the operons that are directly involved in the reactions.

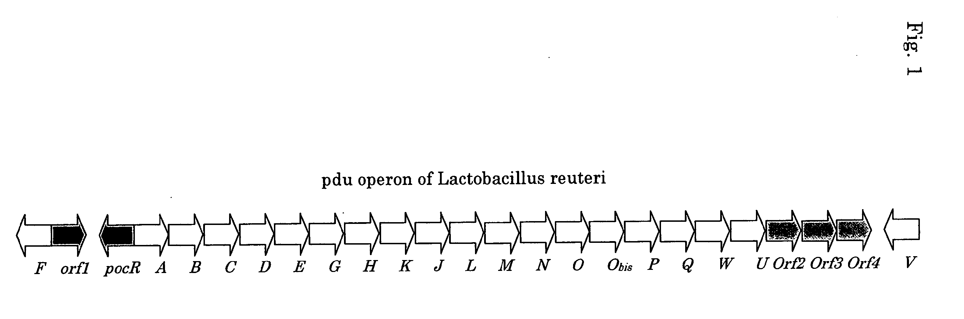

Fig. 1 shows the structure of the pdu operon, and SEQ ID NO: 53 shows the nucleotide sequence of the pdu operon. Functions of each ORF and their nucleotide sequences derived from the *Lactobacillus reuteri* JCM1112 strain are shown below. It is deduced that orf1 to orf4 are not related to the pdu operon. Also, pduObis is considered to have no relationship with the reactions according to the present invention.
- pduF: Accelerator for glycerol ingestion (SEQ ID NO: 54)
- orf1: Ethanolamine utilization EutJ (SEQ ID NO: 55)
- pocR: Transcription regulator (SEQ ID NO: 56)
- pduAB: Polyhedral body structural protein (SEQ ID NOs: 57 and 58)
- pduCDE: Diol dehydratase (SEQ ID NO: 2, 6, 10)
- pduGH: Reactivation factor for diol dehydratase (SEQ ID NOs: 20 and 24)
- pduJK: Polyhedral body structural protein(SEQ ID NOs: 60 and 59)
- pduLM: Unknown (SEQ ID NOs: 61 and 62)
- pduN: Polyhedral body structural protein (SEQ ID NO: 63)
- pduOObis: Adenosyltransferase (SEQ ID NOs: 64 and 65)
- pduP: Propionaldehyde dehydrogenase (SEQ ID NO: 42)
- pduQ: Propanol dehydrogenase (SEQ ID NO: 14)
- pduW: Propionate kinase (SEQ ID NO: 44)
- pduU: Polyhedral body structural protein (SEQ ID NO: 66)
- orf2: Tyrosine phosphatase (SEQ ID NO: 67)
- orf3: Phosphoglycerate mutase (SEQ ID NO: 68)
- orf4: Cadmium resistance (transport protein) (SEQ ID NO: 69)
- pduV: Unknown (SEQ ID NO: 70)

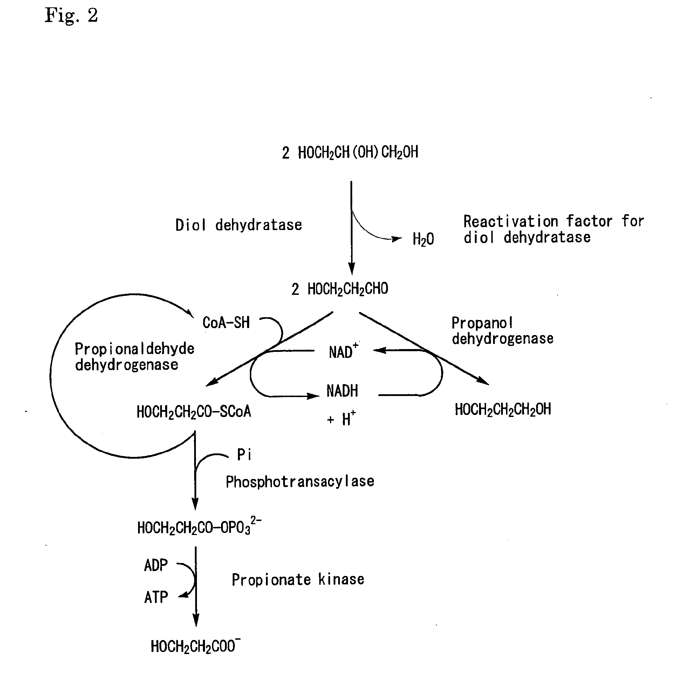

The present inventors discovered that knocking out of the gene encoding glycerol dehydrogenase from bacteria of the genus *Lactobacillus* comprising the pdu operon results in the production of 1,3-propanediol and 3-hydroxypropionic acid from glycerol, based on the mechanism shown in Fig. 2.

In the mechanism shown in Fig. 2, diol dehydratase and the reactivation factor for diol dehydratase can be substituted with glycerol dehydratase and the reactivation factor for glycerol dehydratase, and propanol dehydrogenase can be substituted with 1,3-propanediol oxidoreductase in view of enzyme activities, and similar reactions take place.

Based on such finding, the third aspect of the present invention relates to transformants comprising the gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding propionaldehyde dehydrogenase, the gene encoding phosphotransacylase; the gene encoding propionate kinase; and the gene encoding 1,3-propanediol oxidoreductase and/or the gene encoding propanol dehydrogenase but not comprising any gene encoding glycerol dehydrogenase. The present inventors also discovered that 1,3-propanediol and 3-hydroxypropionic acid could be produced by culturing the transformants according to the third aspect of the present invention in the presence of glycerol.

The transformant according to the third aspect of the present invention comprises a gene encoding a protein having enzyme activity of catalyzing the reaction whereby glycerol is dehydrated and converted into 3-hydroxypropionaldehyde and water. Examples of such protein include glycerol dehydratase and diol dehydratase as mentioned above. The transformant according to the third aspect includes a transformant comprising genes each encoding 3 types of subunits of glycerol dehydratase, a transformant comprising genes each encoding 3 types of subunits of diol dehydratase, and a transformant comprising genes each encoding 3 types of subunits of glycerol dehydratase and genes each encoding 3 types of subunits of diol dehydratase.

The genes each encoding a subunit of glycerol dehydratase or diol dehydratase and the amino acid sequences thereof are as described with respect to the first aspect.

The genes each encoding a subunit may be introduced into the same or different vectors to carry out transformation, as long as such genes are expressed in the same host. It is preferable that three types of subunits be derived from the same species or strain.

The transformant according to the third aspect comprises a gene encoding a protein having enzyme activity of catalyzing a reaction whereby 3-hydroxypropionaldehyde is reduced and converted into propanediol. Examples of genes encoding such proteins include the genes encoding 1,3-propanediol oxidoreductase and the genes encoding propanol dehydrogenase.

The genes encoding propanol dehydrogenase, the genes encoding 1,3-propanediol oxidoreductase, and the amino acid sequences thereof are as described with respect to the first aspect.

The transformant according to the third aspect comprises a gene encoding a protein that replaces coenzyme B12 at the reaction center of glycerol dehydratase or diol dehydratase, which had been deactivated via catalysis of conversion of glycerol into 3-hydroxypropionaldehyde and water, and regains its activity. Examples of such proteins include the reactivation factor for glycerol dehydratase and the reactivation factor for diol dehydratase. The reactivation factor for glycerol dehydratase and the reactivation factor for diol dehydratase are as described with respect to the first embodiment. The transformant according to the third aspect includes a transformant comprising genes each encoding 2 types of subunits of the reactivation factor for glycerol dehydratase, a transformant comprising genes each encoding 2 types of subunits of the reactivation factor for diol dehydratase, and a transformant comprising genes each encoding 2 types of subunits of the reactivation factor for glycerol dehydratase and genes each encoding 2 types of subunits of the reactivation factor for diol dehydratase.

The genes each encoding a subunit of the reactivation factor for glycerol dehydratase or the reactivation factor for diol dehydratase and the amino acid sequences thereof are as described with respect to the first embodiment.

Preferably, a transformant comprising genes each encoding 3 types of subunits of glycerol dehydratase comprises at least genes each encoding 2 types of subunits of the reactivation factor for glycerol dehydratase and a transformant comprising genes each encoding 3 types of subunits of diol dehydratase comprises at least genes each encoding 2 types of subunits of the reactivation factor for the diol dehydratase.

The transformant according to the third aspect comprises a gene encoding a protein having enzyme activity of catalyzing the reaction whereby CoA is added to propionaldehyde to generate propionyl-CoA. An example of genes encoding such proteins is the genes encoding propionaldehyde dehydrogenase. Aldehyde dehydrogenase includes propionaldehyde dehydrogenase.

Known genes encoding propionaldehyde dehydrogenase can be employed. For example, genes derived from bacteria of the genera *Lactobacillus, Citrobacter, Clostridium, Klebsiella, Enterobacter, Caloramator, Salmonella,* and *Listeria* can be employed. In the present invention, the propionaldehyde dehydrogenase gene derived from bacteria of the genus *Lactobacillus* is preferable, the propionaldehyde dehydrogenase gene derived from *Lactobacillus reuteri* is more preferable, and the propionaldehyde dehydrogenase gene derived from the *Lactobacillus reuteri* JCM1112 strain is further preferable.

SEQ ID NO: 41 shows the amino acid sequence of propionaldehyde dehydrogenase derived from *Lactobacillus reuteri,* and SEQ ID NO: 42 shows the nucleotide sequence of the propionaldehyde dehydrogenase gene derived from *Lactobacillus reuteri.* As long as such protein having a amino acid sequence has propionaldehyde dehydrogenase activity, the amino acid sequence as shown in SEQ ID NO: 41 may include mutations such as deletion, substitution, or addition of one or several amino acid residues.

The present invention also includes the use of a gene that hybridizes under stringent conditions with a sequence complementary to DNA comprising part of or the entire nucleotide sequence of DNA comprising the nucleotide sequence as shown in SEQ ID NO: 42 and that encodes a protein having propionaldehyde dehydrogenase activity.

The transformant according to the third aspect comprises a gene encoding a protein having enzyme activity of catalyzing the reaction whereby CoA is removed from propionyl-CoA and phosphoric acid is added to generate propionyl phosphate. An example of a gene encoding such protein is the gene encoding phosphotransacylase.

Known genes encoding phosphotransacylase can be employed. For example, genes derived from bacteria of the genera *Lactobacillus, Citrobacter, Clostridium, Klebsiella, Enterobacter, Caloramator, Salmonella,* and *Listeria* can be employed. In the present invention, the phosphotransacylase gene derived from bacteria of the genus *Lactobacillus* is preferable, the phosphotransacylase gene derived from *Lactobacillus reuteri* is more preferable, and the phosphotransacylase gene derived from the *Lactobacillus reuteri* JCM1112 strain is further preferable.

The transformant according to the third aspect comprises a gene encoding a protein having enzyme activity of catalyzing the reaction whereby phosphoric acid is removed from propionyl phosphate and phosphoric acid is added to ADP to generate ATP and propionic acid simultaneously. An example of gene encoding such protein is the gene encoding propionate kinase.

By a gene having such enzyme activity, ATP is generated simultaneously with the occurrence of the reaction whereby 1,3-propanediol and 3-hydroxypropionic acid are generated, transformants are effectively proliferated, and culture can be effectively carried out.

Known genes encoding propionate kinase can be employed. For example, genes derived from bacteria of the genera *Lactobacillus, Citrobacter, Clostridium, Klebsiella, Enterobacter, Caloramator, Salmonella,* and *Listeria* can be employed. In the present invention, the propionate kinase gene derived from bacteria of the genus *Lactobacillus* is preferable, the propionate kinase gene derived from *Lactobacillus reuteri* is more preferable, and the propionate kinase gene derived from the *Lactobacillus reuteri* JCM1112 strain is further preferable.

SEQ ID NO: 43 shows the amino acid sequence of propionate kinase derived from *Lactobacillus reuteri* and SEQ ID NO: 44 shows the nucleotide sequence of the propionate kinase gene derived from *Lactobacillus reuteri.* As long as such protein having a amino acid sequence has propionate kinase activity, the amino acid sequence as shown in SEQ ID NO: 43 may include mutations such as deletion, substitution, or addition of one or several amino acid residues.

The present invention also includes the use of a gene that hybridizes under stringent conditions with a sequence complementary to DNA comprising part of or the entire nucleotide sequence of DNA comprising the nucleotide sequence as shown in SEQ ID NO: 44 and that encodes a protein having propionate kinase activity.

The transformant according to the third aspect can be obtained by ligating the 4 aforementioned types of genes or parts thereof to an adequate vector and introducing the resulting recombinant vector into a host so as to allow the expression of the gene of the present invention.

The gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding propionaldehyde dehydrogenase; the gene encoding phosphotransacylase; the gene encoding propionate kinase; and the gene encoding 1,3-propanediol oxidoreductase and/or the gene encoding propanol dehydrogenase may be separately introduced into a plurality of vectors to carry out transformation. Alternatively, several types of genes may be introduced into a single vector to carry out transformation.

Vectors for gene introduction are not particularly limited, as long as such vectors can replicate the genes of interest in host cells. Examples thereof include plasmid DNA, phage DNA, and cosmid DNA. Examples of plasmid DNA include pBR322, pSC101, pUC18, pUC 19, pUC118, pUC 119, pACYC117, pBluescript II SK(+), pETDuet-1, and pACYCDuet-1. Examples of phage DNA include λgt10, Charon 4A, M13mp18, and M13mp19.

Any host cells may be employed without particular limitation, as long as the genes of interest can be expressed therein. Examples thereof include bacteria of the genera *Ralstonia, Pseudomonas, Bacillus, Escherichia, Propionibacterium, Lactobacillus, Salmonella, Klebsiella, Acetobacterium, Flavobacterium, Citrobacter, Agrobacterium, Anabaena, Bradyrhizobium, Brucella, Chlorobium, Clostridium; Corynebacterium, Fusobacterium, Geobacter, Gloeobacter, Leptospira, Mycobacterium, Mycobacterium, Photorhabdus, Porphyromonas, Prochlorococcus, Rhodobacter, Rhodopseudomonas, Sinorhizobium, Streptomyces, Synechococcus, Thermosynechococcus,* and *Treponema* and archaebacteria of the genera *Archaeoglobus, Halobacterium, Mesorhizobium, Methanobacterium, Methanococcus, Methanopyrus, Methanosarcina, Methanosarcina, Pyrobaculum, Sulfolobus,* and *Thermoplasma.* Specific examples thereof include *Acetobacterium sp., Citrobacter freundii, Flavobacterium sp., Ralstonia solanacearum, Ralstonia eutropha, Pseudomonas putida, Pseudomonas aeruginosa, Pseudomonas denitrificans, Bacillus subtilis, Bacillus megaterium, Escherichia coli, Propionibacterium acidipropionici, Propionibacterium acnes, Propionibacterium australiense, Propionibacterium avidum, Propionibacterium cyclohexanicum, Propionibacterium granulosum, Propionibacterium jensenii, Propionibacterium microaephilum, Propionibacterium propionicum; Propionibacterium thoenii, Propionibacterium freudenreichii, Agrobacterium tumefaciens, Anabaena sp., Bradyrhizobium japonicum, Brucella melitensis, Brucella suis, Chlorobium tepidum, Clostridium tetani, Clostridium glycolicum, Clostridium difficile, Corynebacterium diphtheriae, Fusobacterium nucleatum, Geobacter sulfurreducens, Gloeobacter violaceus, Leptospira interrogans, Mycobacterium bovis, Mycobacterium tuberculosis, Photorhabdus luminescens, Porphyromonas gingivalis, Prochlorococcus marinus, Rhodobacter capsulatus, Rhodopseudomonas palustris, Sinorhizobium meliloti, Streptomyces avermitilis, Streptomyces coelicolor, Synechococcus sp., Thermosynechococcus elongatus, Treponema denticola, Archaeoglobus fulgidus, Halobacterium sp, Mesorhizobium loti, Methanobacterium Thermoautotrophicum, Methanococcus jannaschii, Methanopyrus kandleri, Methanosarcina acetivorans, Methanosarcina mazei, Pyrobaculum aerophilum, Sulfolobus solfataricus, Sulfolobus tokodaii, Thermoplasma acidophilum,* and *Thermoplasma volcanium.*

Yeast of the genus *Saccharomyces,* such as *Saccharomyces cerevisiae,* yeast of the genus *Candida,* such as *Candida maltosa,* animal cells such as COS cells, CHO cells, mouse L cells, rat GH3 cells, human FL cells, and insect cells, such as SF9 cells, can be employed.

In the third aspect of the present invention, use of bacterial hosts having the system of coenzyme B12 synthesis is preferable. Examples thereof include bacteria of the genera *Lactobacillus, Salmonella, Klebsiella, Propionibacterium, Agrobacterium, Anabaena, Bacillus, Bradyrhizobium, Brucella, Chlorobium, Clostridium, Corynebacterium, Fusobacterium, Geobacter, Gloeobacter, Leptospira, Mycobacterium, Mycobacterium, Photorhabdus, Porphyromonas, Prochlorococcus, Pseudomonas, Ralstonia, Rhodobacter, Rhodopseudomonas, Sinorhizobium, Streptomyces, Synechococcus, Thermosynechococcus,* and *Treponema,* archaebacteria of the genera *Archaeoglobus, Halobacterium, Mesorhizobium, Methanobacterium, Methanococcus, Methanopyrus, Methanosarcina, Methanosarcina, Pyrobaculum, Sulfolobus,* and *Thermoplasma.* Bacteria of the genus *Propionibacterium* is preferable, and *Propionibacterium freudenreichii* is particularly preferable as a host cell. Alternatively, a host cell into which a gene involved in coenzyme B12 synthesis has been introduced via recombination may be used.

Use of host cells wherein glycerol dehydrogenase is not expressed, i.e., a cell that does not contain the glycerol dehydrogenase gene or a cell from which the glycerol dehydrogenase gene is knocked out, is preferable. Use of such cell can block the pathway whereby glycerol is oxidized and converted into dihydroxyacetone. Thus, 1,3-propanediol and 3-hydroxypropionic acid can be produced with higher yield. The glycerol dehydrogenase gene can be knocked out by the same method as described above.

An expression vector, a promoter, and a recombinant vector can be introduced in the same manner as described above.

### Production of 1,3-propanediol and 3-hydroxypropionic acid

In the present invention, 1,3-propanediol and 3-hydroxypropionic acid can be produced in the following manner. That is, the bacteria or transformants of the present invention are brought into contact with glycerol, 1,3-propanediol and 3-hydroxypropionic acid are allowed to accumulate in the reaction product (i.e., the cultured bacteria or culture supernatant), and 1,3-propanediol and 3-hydroxypropionic acid are then recovered.

The condition that "the bacteria or transformants of the present invention be brought into contact with glycerol" refers to culturing of the bacteria or transformants of the present invention in the presence of glycerol or carrying out the reaction with the use of processed culture products of the bacteria or transformants of the present invention. Such processed products include dead bacteria, disrupted bacteria, and crude or purified enzymes prepared from disrupted bacteria or a culture supernatant. Bacteria, processed bacteria, enzymes, or the like, which have been immobilized on carriers via conventional techniques, may also be used.

The bacteria or transformants of the present invention are cultured in accordance with conventional techniques with the use of glycerol as a carbon source. For example, aerobic culture is carried out using a relatively rich medium, such as 2-fold medium, to increase the quantity of bacteria, the atmosphere is converted into anaerobic conditions, and fermentation is then carried out with the addition of glycerol. The pH level is adjusted using a reagent that does not disturb the growth of host cells and that does not block the separation of acid from a fermentation liquor. A sodium carbonate, ammonia, or a sodium ion source such as sodium chloride may be added. General alkaline reagents, such as an aqueous solution of sodium hydroxide, an aqueous solution of potassium hydroxide, an aqueous solution of sodium hydroxide, an aqueous solution of ammonium hydroxide, an aqueous solution of calcium hydroxide, an aqueous solution of potassium carbonate, an aqueous solution of sodium carbonate, or an aqueous solution of potassium acetate, may be used. During the culture, the pH level is maintained between 5.0 and 8.0, and preferably between 5.5 and 7.5.

Examples of nitrogen sources include: ammonia; ammonium salts such as ammonium chloride, ammonium sulfate, and ammonium phosphate; peptone; meat extract; yeast extract; and corn steep liquor. Examples of inorganic materials include monopotassium phosphate, dipotassium phosphate, magnesium phosphate, magnesium sulfate, and sodium chloride.

During the culture, an antibiotic such as kanamycin, ampicillin, or tetracycline may be added to the medium. When a microorganism transformed with an expression vector containing an inducible promoter is cultured, an inducer may be added to the medium. For example, isopropyl-β-D-thiogalactopyranoside (IPTG), indoleacrylic acid (IAA), or the like may be added to the medium.

Transformants obtained by using animal host cells are cultured in medium, such as RPMI-1640, DMEM, or a medium mixture wherein fetal bovine serum is added thereto. Usually, the culture is carried out in the presence of 5% CO2 at 30°C to 40°C for 1 to 30 days. During the culture, an antibiotic such as kanamycin or penicillin may be added to the medium.

Alternatively, the cultured bacteria or transformants obtained above are centrifuged to collect cells and the collected cells are then suspended in an adequate buffer. The resulting cell suspension is suspended in a glycerol-containing buffer, and the resultant is subjected to a reaction to produce 1,3-propanediol and 3-hydroxypropionic acid. The reaction is carried out, for example, at 10°C to 80°C, and preferably at 15°C to 50°C, for 5 minutes to 96 hours, and preferably for 10 minutes to 72 hours, and at pH 5.0 to 8.0, and preferably at pH 5.5 to 7.5.

1,3-Propanediol and 3-hydroxypropionic acid are purified from the culture medium by a method known in the art. For example, 1,3-propanediol and 3-hydroxypropionic acid can be obtained from the culture medium by extraction using an organic solvent or by subjecting the reaction mixture to distillation and column chromatography (US Patent No. 5,356,812). A fermentation liquor is preferably concentrated with the use of a ultrafilter membrane or a zeolite separation membrane that selectively allows permeation of low-molecular weight substances, such as water. Such concentration can reduce the energy required for evaporating water.

The medium may be subjected to high-pressure liquid chromatography (HPLC) analysis to directly identify 1,3-propanediol and 3-hydroxypropionic acid.

The present invention is hereafter described in greater detail with reference to the following examples, although the technical scope of the present invention is not limited thereto.

### Examples

### (Example 1) Acquisition of glycerol dehydratase gene

Synthetic oligonucleotide primers (forward primer: 5'-ATGAAACGTCAAAAACGATTTGAAGAACTAGAAAAAC-3' (SEQ ID NO: 27); and reverse primer: 5'-TTAGTTATCGCCCTTTAGCTTCTTACGACTTT-3' (SEQ ID NO: 28)) were prepared. PCR was carried out using the genome of the *Lactobacillus reuteri* JCM1112 strain as a template under the following conditions.

| Composition of PCR solution (µl) | |
|---|---|
| 10× Buffer KODplus | 5 |
| 2 mM dNTPs | 5 |
| 25 mM MgSO₄ | 2 |
| Genome (111 ng/µl) | 1 |
| KODplus | 1 |
| Water | 34 |
| Forward primer (20 pM) | 1 |
| Reverse primer (20 pM) | 1 |
| Volume of reaction system | total 50 µl |

### Reaction cycle: (94°C for 2 min × 1, 94°C for 15 sec, 45 to 65°C for 30 sec, and 68°C for 5 min) × 30 times, 4°C ∞ (for an indefinite period of time).

Taq Premix was added to the equivalent amount of a solution containing fragments, the mixture was subjected to 3' A-overhanging at 72°C for 10 min, and the purified sample was subjected to TA cloning in pCR4-TOPO. The PRISM 310 and 3100 sequencers (ABI) were used. As a result, the nucleotide sequence of the gene encoding the large subunit of glycerol dehydratase as shown in SEQ ID NO: 2, the nucleotide sequence of the gene encoding the medium subunit of glycerol dehydratase as shown in SEQ ID NO: 6, and the nucleotide sequence of the gene encoding the small subunit of glycerol dehydratase as shown in SEQ ID NO: 10 were determined.

Separately, the forward primer: 5'-ATGAAACGTCAAAAACGTTTTGAAGAACTA-3' (SEQ ID NO: 29) and the reverse primer: 5'-CTAGTTATCACCCTTGAGCTTCTTT-3' (SEQ ID NO: 30) were prepared, and PCR and DNA sequencing were carried out in the same manner as described above using the genome of the *Lactobacillus reuteri* ATCC 53608 strain as a template. As a result, the nucleotide sequence of the gene encoding the large subunit of glycerol dehydratase as shown in SEQ ID NO: 4, the nucleotide sequence of the gene encoding the medium subunit of glycerol dehydratase as shown in SEQ ID NO: 8, and the nucleotide sequence of the gene encoding the small subunit of glycerol dehydratase as shown in SEQ ID NO: 12 were determined.

### (Example 2) Acquisition of propanol dehydrogenase gene

The forward primer: 5'-ATGGGAGGCATAATTCCAATGGAAAFTA-3' (SEQ ID NO: 31) and the reverse primer: 5'-TTAACGAATTATTGCTTCGTAAACCATCTTC-3' (SEQ ID NO: 32) were prepared, and PCR and DNA sequencing were carried out in the same manner as in Example 1 using the genome of the *Lactobacillus reuteri* JCM1112 as a template. As a result, the nucleotide sequence of the propanol dehydrogenase gene as shown in SEQ ID NO: 14 was determined.

Separately, the forward primer: 5'-ATGGGAGGCATAATGCCGATG-3' (SEQ ID NO: 33) and the reverse primer: 5'-TTAACGAATTATTGCTTCGTAAATCATCTTC-3' (SEQ ID NO: 34) were prepared, and PCR and DNA sequencing were carried out in the same manner as in Example 1 using the genome of the *Lactobacillus reuteri* ATCC 53608 strain as a template. As a result, the nucleotide sequence of the propanol dehydrogenase gene as shown in SEQ ID NO: 16 was determined.

### (Example 3) Acquisition of the 1,3-propanediol oxidoreductase gene

The forward primer: 5'-ATGAATAGACAATTTGATTTCTTAATGCCAAG-3' (SEQ ID NO: 35) and the reverse primer: 5'-TTAGTAGATGCCATCGTAAGCCTTTT-3' (SEQ ID NO: 36) were prepared, and PCR and DNA sequencing were carried out in the same manner as in Example 1 using the genome of the *Lactobacillus reuteri* JCM1112 strain as a template. As a result, the nucleotide sequence of the 1,3-propanediol oxidoreductase gene as shown in SEQ ID NO: 18 was determined.

### (Example 4) Acquisition of the gene encoding the reactivation factor for glycerol dehydratase

The forward primer: 5'-ATGGCAACTGAAAAAGTAATTGGTGTTGATATT-3' (SEQ ID NO: 37) and the reverse primer: 5'-TCACCTGTTTGCCATTTCCTTAAAAGGGATT-3' (SEQ ID NO: 38) were prepared, and PCR and DNA sequencing were carried out in the same manner as in Example 1 using the genome of the *Lactobacillus reuteri* JCM1112 strain as a template. As a result, the nucleotide sequence of the gene encoding the large subunit of the reactivation factor for glycerol dehydratase as shown in SEQ ID NO: 20 and the nucleotide sequence of the gene encoding the small subunit of the reactivation factor for glycerol dehydratase as shown in SEQ ID NO: 24 were determined.

Separately, the forward primer: 5'-ATGGCAACTGAAAAAGTAATTGGTGTTG-3' (SEQ ID NO: 39) and the reverse primer: 5'-TCACCTGTTTACCATTTCCTTAAAGG-3' (SEQ ID NO: 40) were prepared, and PCR and DNA sequencing were carried out in the same manner as in Example 1 using the genome of the *Lactobacillus reuteri* ATCC 53608 strain as a template. As a result, the nucleotide sequence of the gene encoding the large subunit of the reactivation factor for glycerol dehydratase as shown in SEQ ID NO: 22 and the nucleotide sequence of the gene encoding the small subunit of the reactivation factor for glycerol dehydratase as shown in SEQ ID NO: 26 were determined.

### (Example 5) Acquisition of the propionaldehyde dehydrogenase gene

The forward primer: 5'-ATGCAGATTAATGATATTGAAAGTGCTGTA-3' (SEQ ID NO: 47) and the reverse primer: 5'-TTAATACCAGTTACGTACTGAGAATCC-3' (SEQ ID NO: 48) were prepared, and PCR and DNA sequencing were carried out in the same manner as in Example 1 using the genome of the *Lactobacillus reuteri* JCM1112 strain as a template. As a result, the nucleotide sequence of the gene encoding propionaldehyde dehydrogenase as shown in SEQ ID NO: 42 was determined.

### (Example 6) Acquisition of the gene encoding propionate kinase

The forward primer: 5'-TTGATGTCAAAAAAAATACTTGCAATTAATTCTG-3' (SEQ ID NO: 49) and the reverse primer: 5'-TTATTGCTGAGTTACATTCATTACATCAC-3' (SEQ ID NO: 50) were prepared, and PCR and DNA sequencing were carried out in the same manner as in Example 1 using the genome of the *Lactobacillus reuteri* JCM1112 strain as a template. As a result, the nucleotide sequence of the gene encoding propionate kinase as shown in SEQ ID NO: 44 was determined.

### (Example 7) Production of 1,3-propanediol and 3-hydroxypropionic acid

### (1) Preparation of recombinant microorganisms

Plasmid 1 comprising the glycerol dehydratase gene of *Lactobacillus* introduced into the multicloning site 1 and the 1,3-propanediol oxidoreductase gene introduced into the multicloning site 2 of the pETDuet-1 vector (Novagen) and plasmid 2 comprising the gene encoding the reactivation factor for glycerol dehydratase gene introduced into the multicloning site 1 and the aldehyde dehydrogenase gene (the aldB gene of *E. coli;* accession number: L40742) introduced into the multicloning site 2 of the pACYCDuet-1 vector (Novagen) were prepared. These plasmids were introduced into BL21(DE3)-RIL via the Gene Pulser II Electroporation System (Bio-Rad).

### (2) Culture of recombinant microorganisms

A 1-µl loopful of the cells was cultured in 5 ml of 2-fold medium containing 100 µg/ml of chloramphenicol and 50 µg/ml of ampicillin (40 g/l of glycerol, 10 g/l of ammonium sulfate, 2 g/l of KH₂PO₄, 6 g/l of K₂HPO₄, 40 g/l of yeast extract, 1 g/l of magnesium sulfate heptahydrate, and 20 drops/l of an antifoaming agent, Adekanol) with shaking at 37°C under aerobic conditions to the late logarithmic growth phase (OD₆₆₀=50). A portion of the culture solution (1 ml) was fractionated, subcultured in 100 ml of 2-fold medium containing 100 µg/ml of chloramphenicol and 50 µg/ml of ampicillin in a 500-ml Sakaguchi flask, and then cultured with shaking at 37°C under aerobic conditions to the late logarithmic growth phase (OD₆₆₀=50).

IPTG was added to a concentration of 1 mM and the mixture was then allowed to stand for 2 hours. The cells were recovered via centrifugation, the recovered cells were added to 100 ml of 1 M glycerol, a 100-ml bottle in which the gas phase has been substituted with nitrogen was allowed to stand on a bottle roller at 37°C for 5 hours, and the liquid was then analyzed. As a result, the liquid was found to contain 0.4 M of 1,3-propanediol and 0.4M of 3-hydroxypropionic acid.

### (Example 8) Production of 1,3-propanediol and 3-hydroxypropionic acid using knockout bacteria

### (1) Preparation of recombinant microorganisms

The ampicillin resistance gene (5 µg, SEQ ID NO: 73) comprising the sequence 5'-ATGGACCGCATTATTCAATCACCGGGTAAATACATCCAGGGCGCTGATGTGATTAAT CGTTAACC-3' (primer 1: SEQ ID NO: 71) at its N-terminus and the sequence 5'-CTGGGCGAATACCTGAAGCCGCTGGCAGAACGCTGGTTAGTGGTGGGTGACAAAT TTG-3' (primer 2: SEQ ID NO: 72) was mixed with 50 µl of a solution containing *E. coli* Top 10 electrocompetent cells, and the resulting mixture was transferred to a 0.1 cm cuvette using a Gene-Pulser II (Bio-Rad). The Gene-Pulser II was set at 2.0 kV, 25 mF, and 200 Ω, and electrical pulses were applied. The pulsed mixture was introduced into 250 µl of SOC medium, the mixture was cultured at 37°C for 1 hour, the culture product was applied onto an agar plate of LB medium containing 50 µg/ml of ampicillin, and ampicillin resistance strains were selected. The ampicillin resistance strains were subjected to colony PCR using the primer 1 and the primer 2, and strains exhibiting amplification of fragments of approximately 2300 bp were designated as the glycerol dehydrogenase gene knockout strains.

The genome of *Klebsiella* oxytoca ATCC8724 (1 µg) was processed with 1U of Sau3AI restriction enzyme at 37°C for 10 minutes, and the processed sample was separated via electrophoresis to recover and purify DNA at around 25 to 35 kb. The purified DNA was ligated to a Charomid 9-20 vector (Nippon Gene), the product was packaged using Lambda INN (Nippon Gene), and the packaged product was caused to infect the E. *coli* TOP10 competent cells, from which the glycerol dehydrogenase genes were knocked out, for transformation. From among the resulting transformants, probe 1 (SEQ ID NO: 74), which is a conserved region in the ORF of pocR located at the anterior end of any type of pdu operon, and probe 2 (SEQ ID NO: 75), which is a conserved region in the ORF of pduV located at the posterior end of any type of pdu operon, were selected, colony hybridization was carried out, and strains positive for both probes were selected.

### (2) Culture of recombinant microorganisms

A 1-µl loopful of the cells was cultured in 5 ml of 2-fold medium containing 100 µg/ml of chloramphenicol and 50 µg/ml of ampicillin (40 g/l of glycerol, 10 g/l of ammonium sulfate, 2 g/l of KH₂PO₄, 6 g/l of K₂HPO₄, 40 g/l of yeast extract, 1 g/l of magnesium sulfate heptahydrate, and 20 drops/l of an antifoaming agent, Adekanol) with shaking at 37°C under aerobic conditions to the late logarithmic growth phase (OD₆₆₀=50). A portion of the culture solution (1 ml) was fractionated, subcultured in 100 ml of 2-fold medium containing 100 µg/ml of chloramphenicol and 50 µg/ml of ampicillin in a 500-ml Sakaguchi flask, and then cultured with shaking at 37°C under aerobic conditions to the late logarithmic growth phase (OD₆₆₀=50).

IPTG was added to a concentration of 1 mM and the mixture was then allowed to stand for 2 hours. The cells were recovered via centrifugation, the recovered cells were added to 200 ml of 1M glycerol, a 100-ml bottle in which the gas phase has been substituted with nitrogen was allowed to stand on a bottle roller at 37°C for 5 hours, and the liquid was then analyzed. As a result, the liquid was found to contain 25 mM of 1,3-propanediol and 21 mM of 3-hydroxypropionic acid.

### (Example 9) Production of 1,3-propanediol and 3-hydroxypropionic acid using knockout bacteria

In order to disrupt the glycerol dehydrogenase gene, the sequence of the glycerol dehydrogenase gene (SEQ ID NO: 45) was analyzed, the KpnI site at around 830 bp from the initiation codon was selected, and a drug resistance marker was introduced into the restriction enzyme site in order to confirm the introduction of gene disruption.

### (1) Acquisition of glycerol dehydrogenase of Lactobacillus reuteri JCM1112 strain and introduction thereof into pCR4-TOPO vector

Based on the genomic information, the following primers for amplifying the glycerol dehydrogenase gene of the *Lactobacillus reuteri* JCM1112 strain were prepared.
Forward primer: 5'-ATGGTTGAAGAATTTGGCTCACC-3' (SEQ ID NO: 51)
Reverse primer: 5'-TTACATACGACTATGGTGACAACG-3' (SEQ ID NO: 52)

As a result of amplification by PCR, a 1112-bp fragment of interest was obtained. The resultant was subjected to 3'A-overhanging, further purification, TA cloning using the Invitrogen TOPO TA cloning kit, and then transformation into the TOP10 cells. A plasmid (pCR4-TOPO/Lb_GDH) was recovered from a transformant having a plasmid comprising the glycerol dehydrogenase gene of the *Lactobacillus reuteri* JCM1112 strain introduced into the TA cloning site of the pCR4-TOPO vector.

### (2) Preparation of drug resistance marker gene and processing thereof with restriction enzyme

The sequence of the pIL253 plasmid, which has been disclosed on the Internet (see http://www. ncbi.nlm.nih.gov/entrez/viewer.fcgi?db=nucleotide&val=277339), was analyzed, and a DNA fragment as shown in SEQ ID NO: 46 was synthesized so as to comprise the erythromycin resistance gene that can be used as a drug resistance marker in *Lactobacillus,* a promoter thereof, a terminator region, and KpnI sites at both ends. The solution having the composition shown in Table 1 was used to subject this DNA fragment to processing with a restriction enzyme at 37°C for 2 hours, and the DNA fragment was purified, recovered, and then designated as a drug resistance marker sequence.

**Table 1**

| | |
|---|---|
| 10x Buffer L | 10 µl |
| Synthesized DNA sequence (amount of DNA: 1 µg) | 30 µl |
| KpnI | 10 µl |
| Purified water | 50 µl |
| Total | 100 µl |

### (3) Preparation of a fragment for introducing gene disruption

### a) Preparation of linearized pCR4-TOPO/Lb_GDH

The solution having the composition shown in Table 2 was used to perform restriction enzyme treatment at 37°C for 2 hours and a linearized plasmid of approximately 4986 bp was recovered.

**Table 2**

| | |
|---|---|
| 10x Buffer L | 10 µl |
| PCR4-TOPO/Lb_GDH | 50 µl |
| KpnI | 10 µl |
| Purified water | 30 µl |
| Total | 100 µl |

Subsequently, the solution having the composition shown in Table 3 was used to subject the linearized pCR4-TOPO/Lb_GDH to alkaline phosphatase treatment at 37°C for 1.5 hours, followed by purification and recovery. The concentration of the recovered linearized pCR4-TOPO/Lb_GDH was 30 ng/µl.

**Table 3**

| | |
|---|---|
| 10x BAP buffer | 10 µl |
| pCR4-TOPO/Lb_GDH processed with restriction enzyme | 50 µl |
| BAP (2.5 U) | 10 µl |
| Purified water | 30 µl |
| Total | 100 µl |

### b) Preparation of pCR-4/TOPO vector having a fragment for introducing gene disruption as insertion sequence

The linearized plasmid prepared in a) was ligated to the drug resistance marker sequence prepared in 2), and the ligation product was transformed into the E. *coli* TOP10 cells. A pCR4-TOPO plasmid (hakai/ pCR4-TOPO) having a fragment for introducing gene disruption as an insertion sequence was selected and then recovered.

### c) Preparation of a fragment for introducing gene disruption

PCR was carried out using the hakai/pCR4-TOPO prepared in b) as a template and the primers used in 1),
Forward primer: 5'-ATGGTTGAAGAATTTGGCTCACC-3' (SEQ ID NO: 51)
Reverse primer: 5'-TTACATACGACTATGGTGACAACG-3' (SEQ ID NO: 52)
and a target fragment of 2144 bp was amplified.

A large portion of the resulting fragment was purified, the resultant was concentrated to an average concentration of 5 µg/µl, and the resultant was used in the experiment for introducing gene disruption.

### (4) Introduction of gene disruption

### a) Preparation of competent cells

Competent cells were prepared in the following manner.
1. MRS media (10 ml×5) each were inoculated with 100 ml of L. *reuteri* solution that had been cultured overnight, and culture was continued to result in OD₆₆₀ ≒ 0.8. (culture was performed in a test tube, and anaerobic culture was performed in a gas-pack system for about 5 to 5.5 hours).
2. Five tubes of culture solution were transferred to 50-ml falcon tubes, and cells were collected, followed by washing three times with 30 to 40 ml of sterile distilled water at room temperature.
3. The cells were suspended in 800 ml of sterile distilled water (room temperature), transferred to a 1.5-ml microtube, and then collected.
4. The supernatant was discarded, and the remnant was suspended in 250 ml of 30% (wt/vol) PEG1500 (dH₂O).
5. The solution was fractionated to fractions of 100 ml each and preserved at -80°C.
6. The product was dissolved immediately before use and then subjected to electroporation.

### b) Introduction of a fragment for introducing gene disruption into competent cell

1. The test plasmid (5 to 10 ml; 2 to 3 mg) was added to the competent cells prepared in the above-described manner.
2. The resulting mixture was transferred to a 0.2-cm cuvette that had been cooled in advance.
3. The Gene pulser was set at 2.5 kV, 25 mF, and 200 Ω, and electropulses were applied.
4. Immediately thereafter, MRS broth that had been heated at 37°C in advance was added to result in a total amount of 1 ml, and the mixture was then incubated at 37°C for 1.5 to 2 hours.
5. The incubation product was applied to the MRS agar containing erythromycin (2 mg/ml), and anaerobic culture was performed at 37°C for 24 to 48 hours (anaerobic culture was performed in a gas-pack system).

### c) Selection of disrupted strain

The erythromycin resistance strain that had grown as a result of culture in b) above was selected, and the genome thereof was recovered. PCR was carried out using the recovered genome as a template to confirm the presence of the insert. The strain, drug resistance of which and gene introduction into the genome were confirmed by PCR, was designated as a disrupted strain.

### (5) Reaction using disrupted strain

To 10 ml of medium comprising common MRS medium and 2% glycerin, 100 µl of a solution containing the disrupted strains that had been cultured overnight was added, and the resultant was cultured at 37°C for 24 hours under anaerobic conditions. The cells were recovered by centrifugation at 2500xg for 10 minutes, the recovered cells were washed two times with 10 ml of 50 mM potassium phosphate buffer (pH 7.5), 10 ml of 50 mM potassium phosphate buffer (pH 7.5) containing 2% glycerin was added thereto, and the reaction was allowed to proceed at 37°C. The reaction results are as shown below. As a control example, nondisrupted *Lactobacillus* cells were used. The results are shown in Table 4.

**Table 4**

| Reaction results of disrupted strain | | | | | | |
|---|---|---|---|---|---|---|
| Reaction time (hr) | 0 | 24 | 48 | 72 | 96 | 120 |
| Glycerol (mM) | 200 | 140 | 100 | 50 | 25 | 0 |
| 1,3-Propanediol (mM) | 0 | 29 | 48 | 72 | 85 | 95 |
| 3-Hydroxypropionic acid (mM) | 0 | 27 | 47 | 70 | 83 | 94 |

| Reaction results of wild strain | | | | | | |
|---|---|---|---|---|---|---|
| Reaction time (hr) | 0 | 24 | 48 | 72 | 96 | 120 |
| Glycerol (mM) | 200 | 180 | 181 | 180 | 180 | 180 |
| 1,3-Propanediol (mM) | 0 | 10 | 9 | 10 | 9 | 12 |
| 3-Hydroxypropionic acid (mM) | 0 | 0 | 0 | 0 | 0 | 0 |

As is apparent from the foregoing description, the present invention can reduce loss in starting glycerol used when producing 1,3-propanediol from glycerol and can produce 1,3-propanediol and 3-hydroxypropionic acid.

### (Example 10) Confirmation of phosphotransacylase activity

The cell broth at the late logarithmic growth phase (10 ml, the *Lactobacillus reuteri* JCM1112 strain) was washed two times with 10 ml of 50 mM potassium phosphate buffer (pH 8) and resuspended in 10 ml of 50 mM potassium phosphate buffer (pH 8), followed by disruption of cells via ultrasonication with ice cooling. The product was centrifuged at 20,000× g for 30 minutes, and the supernatant was designated as a crude enzyme solution. The crude enzyme solution was adequately diluted, the dilution was added to a buffer comprising acetyl-CoA (pH 7.5), the reaction was allowed to proceed at 37°C, and generation of CoA was investigated. As a result, generation of CoA was confirmed.

The genome of the *Lactobacillus reuteri* JCM1112 strain was analyzed, and ORF that was homologous to phosphotransacylase was observed.

### Sequence Listing Free Text

SEQ ID NOs: 27 to 40 and 46 to 52: Synthetic oligonucleotides

## Claims

1. A transformant comprising the gene encoding the large subunit of glycerol dehydratase and/or diol dehydratase, the gene encoding the medium subunit thereof, and the gene encoding the small subunit thereof; the gene encoding the large subunit of the reactivation factor for glycerol dehydratase and/or the reactivation factor for diol dehydratase and the gene encoding the small subunit thereof; the gene encoding aldehyde dehydrogenase; and the gene encoding 1,3-propanediol oxidoreductase and/or the gene encoding propanol dehydrogenase.

2. The transformant according to claim 1, wherein the genes each encoding a subunit of glycerol dehydratase and/or diol dehydratase are derived from *Lactobacillus reuteri.*

3. The transformant according to claim 1 or 2, which comprises the gene encoding propanol dehydrogenase and said gene is derived from *Lactobacillus reuteri.*

4. The transformant according to any of claims 1 to 3, which comprises the gene encoding 1,3-propanediol oxidoreductase and said gene is derived from *Lactobacillus reuteri.*

5. The transformant according to any of claims 1 to 4, wherein the genes each encoding a subunit of the reactivation factor for glycerol dehydratase and/or the reactivation factor for diol dehydratase are derived from *Lactobacillus reuteri.*

6. The transformant according to claim 1, wherein the genes encoding aldehyde dehydrogenase is genes encoding propionaldehyde dehydrogenase, and said transformant further comprises the genes encoding phosphotransacylase and the genes encoding propionate kinase but does not comprise any gene encoding glycerol dehydrogenase.

7. The transformant according to claim 6, which comprises the pdu operon and no gene encoding glycerol dehydrogenase.

8. Knockout bacteria, which are obtained by knocking out the gene encoding glycerol dehydrogenase from bacteria of the genera *Lactobacillus, Salmonella, Klebsiella, Listeria, Clostridium, Escherichia, Enterobacter, Caloramator, Acetobacterium, Brucella, Flavobacterium, Fusobacterium, Citrobacter,* or *Propionibacterium.*

9. Knockout bacteria comprising the pdu operon and the gene encoding phosphotransacylase, wherein the gene encoding glycerol dehydrogenase is knocked out.

10. A method for producing 1,3-propanediol and/or 3-hydroxypropionic acid by bringing the transformants or bacteria according to any one of claims 1 to 9 into contact with glycerol.
